# FASCICULE DE BREVET EUROPEEN

(11) **EP 1 077 932 B1**
(45) Date de publication et mention de la délivrance du brevet: **16.07.2003**
(21) Numéro de dépôt: 99918054.0
(22) Date de dépôt: 11.05.1999
(51) Int. Cl.: C07C 253/30, C07C 209/48

(54) **PROCEDE DE PREPARATION D'AMINONITRILE ET DE DIAMINE**
VERFAHREN ZUR HERSTELLUNG EINES AMINONITRILS UND EINES DIAMINS
METHOD FOR PREPARING AMINONITRILE AND DIAMINE

(30) Priorité: 15.05.1998 FR 9806426
(43) Date de publication de la demande: 28.02.2001
(73) Titulaire: Rhodia Polyamide Intermediates, 69192 Saint-Fons (FR)
(72) Inventeur: LECONTE, Philippe, F-69330 Meyzieu (FR)
(74) Mandataire: Esson, Jean-Pierre
(86) Numéro de dépôt international: FR9901127
(87) Numéro de publication internationale: WO99059962

(56) Documents cités:
- WO-A-95/18090
- WO-A-96/18603
- WO-A-98/11060
- FR-A- 2 722 709

## Description

La présente invention concerne un procédé de préparation d'aminonitrile et de diamine par hydrogénation catalytique de dinitrile.

Elle concerne plus particulièrement la préparation d'amino-6 capronitrile et d'hexaméthylènediamine par hydrogénation catalytique de l'adiponitrile.

L'hydrogénation catalytique d'un dinitrile aliphatique, notamment l'adiponitrile, en aminonitrile et diamine, notamment l'amino-6 capronitrile et l'hexaméthylènediamine, peut être réalisée par exemple selon l'enseignement du brevet WO-A-96/18603 en présence d'un catalyseur à base de nickel de Raney ou de cobalt de Raney, contenant éventuellement un ou plusieurs éléments promoteurs, et en présence d'une base minérale forte, en opérant dans un milieu contenant outre le dinitrile, de l'eau, de la diamine et/ou de l'aminonitrile.

On peut également se référer au brevet EP-A-0 737 100 qui décrit l'hydrogénation d'un dinitrile aliphatique, notamment l'adiponitrile, en présence d'un nickel ou d'un cobalt de Raney dopé chimiquement par un ou plusieurs éléments promoteurs, et en présence d'une base forte, en opérant dans un milieu contenant au moins de l'eau et le cas échéant un solvant organique tel qu'un alcool ou un amide.

Le brevet WO-A-97/10052 décrit l'hydrogénation de nitriles, notamment de l'adiponitrile, en présence d'un catalyseur à base de nickel ou de cobalt au moins en partie à l'état réduit, texturé par une phase comprenant un ou plusieurs métaux dopants sous forme d'oxydes, et en présence d'une base forte, en opérant dans un milieu contenant au moins de l'eau et le cas échéant un solvant organique tel qu'un alcool ou un amide.

Le brevet WO 9811060 décrit l'hydrogénation partielle d'adiponitrile en présence d'un catalyseur, l'amino-b-capronitrile et l'hexaméthylène diamine ainsi formées sont séparées par distillation et la fraction contenant l'adiponitrile non transformée est ensuite acidifiée, séparée et recyclée.

Dans ces procédés de l'art antérieur, le mélange réactionnel final est dosé et les teneurs en impuretés éventuelles sont déterminées. Les dites teneurs d'impuretés sont généralement faibles, voire nulles.

Cependant il est bien évidemment nécessaire de procéder à la séparation des constituants dudit mélange réactionnel, essentiellement l'eau, le solvant éventuel, les autres composés légers éventuellement présents, la diamine et l'aminonitrile formés ainsi que le dinitrile non transformé.

La demanderesse a observé que lors de la distillation du mélange réactionnel, après séparation du catalyseur par filtration, décantation, centrifugation ou tout autre moyen, il se forme des quantités importantes et prohibitives de sous-produits provenant notamment de la dégradation du dinitrile, notamment de l'adiponitrile. Sans que cela soit limitatif, lorsque le dinitrile mis en oeuvre est l'adiponitrile, le principal de ces sous-produits est l'iminocyanocyclopentane (ICCP), avec d'autres sous-produits plus lourds.

La formation des sous-produits présente de nombreux inconvénients et il est par conséquent nécessaire de l'éviter le plus possible. Tout d'abord ces sous-produits proviennent essentiellement de la transformation du dinitrile, en particulier l'adiponitrile, ce qui occasionne une perte qui ne peut pas être négligée dans les procédés industriels. D'autre part, ils induisent des colorations et/ou ils ont une influence néfaste considérable sur le niveau des spécifications auxquelles doivent répondre par exemple aussi bien l'hexaméthylènediamine pour la préparation de polyamide-66 que l'amino-6 capronitrile qui conduit au caprolactame, lui-même matière de base du polyamide-6.

En outre, ces sous-produits sont difficiles à éliminer, ce qui nécessite des procédés de purification complexes et coûteux.

La présente invention a pour objet de remédier à ces inconvénients importants en réduisant le plus possible la formation de tels sous-produits au cours de la distillation des constituants des mélanges réactionnels issus de l'hydrogénation catalytique des dinitriles, en particulier des dinitriles aliphatiques.

Elle consiste en un procédé de préparation d'aminonitrile et de diamine par hydrogénation catalytique de dinitrile aliphatique ayant de 3 à 12 atomes de carbone, en présence éventuellement d'un solvant caractérisé en ce que le mélange réactionnel final, dont le catalyseur a été préalablement séparé, est acidifié par addition d'une quantité suffisante d'un acide minéral ou organique, avant d'être soumis à une opération de distillation des produits de la réaction et du dinitrile non transformé.

Parmi les dinitriles, on peut plus particulièrement citer l'adiponitrile, le méthylglutaronitrile, l'éthylsuccinonitrile, le diméthylsuccinonitrile, le malononitrile, le succinonitrile, le glutaronitrile, le dodécanedinitrile. On peut mettre en oeuvre des mélanges de plusieurs dinitriles, notamment des mélanges comprenant de l'adiponitrile, du méthylglutaronitrile et de l'éthylsuccinonitrile, provenant de la synthèse d'adiponitrile à partir du butadiène.

Par commodité, le procédé de l'invention se référera généralement dans ce qui suit à l'adiponitrile et à ses produits d'hydrogénation, l'amino-6 capronitrile et l'hexaméthylènediamine, mais il s'applique également aux autres dinitriles.

Le procédé de l'invention s'applique plus particulièrement aux mélanges provenant de l'hydrogénation de dinitriles catalysée par au moins un métal du groupe VIII de la Classification périodique des éléments telle que publiée dans "Handbook of Chemistry and Physics, 51st Edition (1970-1971)" de The Chemical Rubber Company.

Plus particulièrement, l'hydrogénation de l'adiponitrile est effectuée de manière connue. Les catalyseurs mis en oeuvre comprennent le plus souvent au moins un métal choisi parmi le nickel, le cobalt: le fer. le rhodium, le ruthénium. Ces métaux peuvent être associés à un ou plusieurs éléments promoteurs. Comme éléments promoteurs, on peut citer par exemple le molybdène, le tungstène, le titane, le chrome, le fer, le nickel, le cobalt, le cuivre, l'argent, l'or, le zinc, le cadmium, le plomb, l'étain, le palladium, le platine, l'osmium, le rhénium, l'iridium, l'antimoine, le bismuth, les métaux des terres rares.

Le catalyseur peut être ou non déposé sur un support. On peut utiliser comme supports l'alumine, la silice, le dioxyde de titane, le dioxyde de zirconium, l'oxyde de magnésium, les charbons actifs.

Parmi les catalyseurs non supportés, on préfère le nickel de Raney et le cobalt de Raney, comportant éventuellement un ou plusieurs éléments promoteurs. Parmi les promoteurs plus particulièrement adaptés au nickel de Raney ou au cobalt de Raney, on peut citer le titane, le molybdène, le tungstène, le chrome, le fer, le zinc, le cuivre, l'argent, l'or.

On peut également utiliser les catalyseurs à base de nickel ou de cobalt au moins en partie à l'état réduit, texturé par une phase comprenant un ou plusieurs métaux dopants (promoteurs) sous forme d'oxydes, tels que décrits dans le brevet WO-A-97/10052

Pour l'hydrogénation de l'adiponitrile en présence de nickel de Raney ou de cobalt de Raney, on peut se référer par exemple aux brevets WO-A-96/18603, EP-A-0 737 100, EP-A-0 737 101 ou EP-A-0 737 181.

L'hydrogénation de l'adiponitrile et plus particulièrement lorsqu'elle est opérée en présence de nickel de Raney, de cobalt de Raney, de rhodium ou de ruthénium, est de préférence conduite en présence d'un composé basique tel qu'un hydroxyde de métal alcalin ou alcalino-terreux.

La réaction d'hydrogénation est généralement conduite en présence de solvant tel que l'eau. On peut opérer en outre en présence d'un solvant organique tel qu'un alcool ou un amide ou d'un solvant minéral tel que l'ammoniac liquide. Le milieu réactionnel peut également comprendre dès le début de la réaction des quantités variables des produits de ladite réaction jouant le rôle de solvants.

Le solvant est séparé du milieu réactionnel, préférentiellement après neutralisation de la base présente dans le milieu, notamment quand celle-ci est une base minérale.

L'acide additionné au mélange réactionnel après séparation du catalyseur, peut être tout acide minéral tel que par exemple l'acide sulfurique, l'acide phosphorique, l'acide phosphoreux, l'acide chlorhydrique, l'acide nitrique, ou organique tel que par exemple les acides carboxyliques aliphatiques, cycloaliphatique, aromatiques, mono ou polyfonctionnels ou les acides sulfoniques aliphatiques, cycloaliphatiques ou aromatiques. A titre d'exemples non limitatifs d'acides organiques, on peut citer l'acide acétique, l'acide propionique, l'acide valérique, l'acide hexanoïque, l'acide adipique, l'acide téréphtalique, l'acide glutarique, l'acide succinique, l'acide méthylglutarique, l'acide éthylsuccinique, l'acide paratoluènesulfonique, l'acide méthanesulfonique, l'acide fluorométhanesulfonique. On peut également utiliser des résines acides notamment des résines à groupements sulfoniques.

La quantité d'acide mise en oeuvre doit en général être telle qu'elle corresponde au moins à la stoechiométrie par rapport aux composés basiques minéraux présents dans le mélange réactionnel issu de l'hydrogénation du dinitrile, plus particulièrement de l'adiponitrile. En général on ajoute de 0,0005 % à 2 % en poids d'acide par rapport au poids du mélange réactionnel et de préférence de 0,001 % à 1 % en poids par poids.

Lorsque l'acide utilisé est une résine acide, le rapport pondéral n'a guère de signification. On peut soit passer le mélange réactionnel à traiter sur ladite résine, soit le cas échéant introduire de la résine dans le mélange avant sa distillation.

Après l'ajout d'acide le mélange réactionnel est distillé afin de séparer le solvant et les autres composés légers qu'il peut contenir, l'hexaméthylènediamine et l'amino-6 capronitrile formés ainsi que l'adiponitrile non transformé qu'il contient et qui peut être recyclé dans une nouvelle hydrogénation. La distillation de l'hexaméthylènediamine et de l'amino-6 capronitrile peut être effectuée successivement dans deux colonnes ou être dans un premier temps effectuée globalement afin de chauffer moins longtemps l'adiponitrile non transformé. Dans la deuxième variante, l'hexaméthylènediamine et l'amino-6 capronitrile sont séparés ensuite l'un de l'autre par distillation. L'adiponitrile peut être recyclé directement dans une opération d'hydrogénation ou être préalablement lui-même distillé pour éliminer les produits lourds qu'il peut contenir, notamment les sels formés par l'acide ajouté. L'amino-6 capronitrile peut être hydrolysé en phase liquide ou en phase gazeuse pour conduire au caprolactame. Cette hydrolyse est effectuée selon les techniques connues, en présence ou non d'un catalyseur et éventuellement après une purification complémentaire de l'amino-6 capronitrile. Le caprolactame conduit par polymérisation au polyamide-6. L'hexaméthylènediamine peut servir tout particulièrement à préparer le polyamide-66 par réaction avec l'acide adipique.

Lorsque les composés légers ont été séparés, la distillation est généralement effectuée sous pression inférieure à la pression atmosphérique.

Par comparaison avec une distillation effectuée sans ajout préalable d'acide ou avec un ajout d'acide effectué après séparation de l'amino-6 capronitrile et de l'hexaméthylènediamine, la quantité de sous-produits formés au cours de la distillation est considérablement diminuée.
Les exemples qui suivent illustrent l'invention.

### EXEMPLE 1

On réalise l'hydrogénation continue d'adiponitrile à 50°C sous 20 bar d'hydrogéne en présence de 15 % en poids par rapport au mélange réactionnel de nickel de Raney (comportant 1,8 % en poids de Cr par rapport au Ni) et de potasse à raison d'environ 0,42 % en poids par rapport au mélange réactionnel.

Le mélange réactionnel a la composition pondérale suivante : 27 % d'hexaméthylènediamine (HMD), 38 % d'amino-6 capronitrile (ACN), 25 % d'adiponitrile (AdN) et 9 % d'eau et il contient en outre 0,0080 % de potasse.

Par dosage en chromatographie en phase gazeuse, on ne détecte pas de présence d'iminocyanocyclopentane (ICCP).

A 450 g de ce mélange, on ajoute 63 mg d'acide orthophosphorique (environ 1 mol par mol de potasse).

On distille ensuite l'eau sous pression atmosphérique en 1 h.

On distille ensuite sous pression réduite l'ensemble HMD/ACN en 2 h jusqu'à une température dans le bouilleur de 185 °C.

On dose alors l'ICCP sur un échantillon du pied de distillation (AdN) : on trouve 0,003 % d'ICCP.

Afin de simuler une opération continue pendant laquelle le pied de distillation a un temps de séjour plus important, ledit pied de distillation est maintenu encore pendant 2 h à 185 °C.

On effectue un dosage d'ICCP sur le pied de distillation ainsi traité : on trouve alors 0,010 % d'ICCP.

### ESSAI COMPARATIF 1

Cet essai comparatif est effectué sur 450 g du mélange préparé par hydrogénationde l'AdN dans la première partie de l'exemple 1.

On distille ensuite l'eau sous pression atmosphérique en 1 h.

On distille ensuite sous pression réduite l'ensemble HMD/ACN en 2 h jusqu'à une température dans le bouilleur de 185 °C.

On dose alors l'ICCP sur un échantillon du pied de distillation (AdN) : on trouve 12 % d'ICCP et on constate que 23 % de l'AdN présent dans le mélange initial ont disparu : transformation en ICCP d'une part et en produits lourds (point d'ébullition supérieur à celui de l'AdN), d'autre part.

En absence de traitement par un acide avant la distillation des constituants du mélange, on observe donc la formation d'énormément plus d'ICCP que dans le cadre de l'invention avec en outre l'apparition de quantités très importantes de produits lourds non valorisables, au détriment de l'AdN présent dans le mélange d'origine.

On rajoute au culot de distillation précédent 63 mg d'acide orthophosphorique (même quantité que dans l'exemple 1).

On distille alors l'AdN sous pression réduite pendant 1 h.

La quantité d'ICCP et de composés lourds restant dans le bouilleur est sensiblement la même que la quantité dosée avant addition de l'acide phosphorique.

## Revendications

1. Procédé de préparation d'aminonitrile et de diamine par hydrogénation catalytique de dinitrile aliphatique ayant de 3 à 12 atomes de carbone, en présence éventuellement d'un solvant, **caractérisé en ce que** le mélange réactionnel final, dont le catalyseur a été préalablement séparé, est acidifié par addition d'une quantité suffisante d'un acide minéral ou organique, avant d'extraire par distillation les produits de la réaction et le dinitrile non transformé.

2. Procédé selon la revendication 1, **caractérisé en ce qu'**il s'applique aux mélanges provenant de l'hydrogénation de dinitriles catalysée par au moins un métal du groupe VIII de la Classification périodique des éléments.

3. Procédé selon l'une des revendications 1 ou 2, **caractérisée en ce qu'**il est appliqué aux mélanges réactionnels provenant de la préparation d'amino-6 capronitrile et d'hexaméthylènediamine par hydrogénation catalytique d'adiponitrile.

4. Procédé selon l'une des revendications 1 à 3, **caractérisé en ce que** l'hydrogénation est effectuée en présence de catalyseurs comprenant au moins un métal choisi parmi le nickel, le cobalt; le fer, le rhodium, le ruthénium, éventuellement associé à un ou plusieurs éléments promoteurs.

5. Procédé selon la revendication 4, **caractérisé en ce que** les éléments promoteurs sont choisis parmi le molybdène, le tungstène, le titane, le chrome, le fer, le nickel, le cobalt, le cuivre, l'argent, l'or, le zinc, le cadmium, le plomb, l'étain, le palladium, le platine, l'osmium, le rhénium, l'iridium, l'antimoine, le bismuth, les métaux des terres rares.

6. Procédé selon l'une des revendications 1 à 5, **caractérisé en ce que** le catalyseur est déposé sur un support choisi parmi l'alumine, la silice, le dioxyde de titane, le dioxyde de zirconium, l'oxyde de magnésium, les charbons actifs.

7. Procédé selon l'une des revendications 1 à 5, **caractérisé en ce que** le catalyseur est choisi parmi le nickel de Raney et le cobalt de Raney, comportant éventuellement un ou plusieurs éléments promoteurs, tels que le titane, le molybdène, le chrome, le fer, le tungstène, le zinc, le cuivre, l'argent, l'or.

8. Procédé selon l'une des revendications 1 à 5, **caractérisé en ce que** le catalyseur est choisi parmi les catalyseurs à base de nickel ou de cobalt au moins en partie à l'état réduit, texturé par une phase comprenant un ou plusieurs métaux promoteurs sous forme d'oxydes.

9. Procédé selon l'une des revendications 1 à 8, **caractérisé en ce que** l'acide additionné au mélange réactionnel après séparation du catalyseur est un acide minéral tel que l'acide sulfurique, l'acide phosphorique, l'acide phosphoreux, l'acide chlorhydrique, l'acide nitrique, ou organique tel que les acides carboxyliques aliphatiques, cycloaliphatiques, aromatiques, mono ou polyfonctionnels ou les acides sulfoniques aliphatiques, cycloaliphatiques ou aromatiques, ou une résine acide, de préférence à groupements sulfoniques.

10. Procédé selon l'une des revendications 1 à 9, **caractérisé en ce que** la quantité d'acide mise en oeuvre est telle qu'elle correspond au moins à la stoechiométrie par rapport aux composés basiques minéraux présents dans le mélange réactionnel issu de l'hydrogénation du dinitrile.

11. Procédé selon l'une des revendications 1 à 10, **caractérisé en ce que** la quantité d'acide mise en oeuvre représente de 0,0005 % à 2 % en poids d'acide par rapport au poids du mélange réactionnel et de préférence de 0,001 % à 1 % en poids par poids.

12. Procédé selon l'une des revendications précédentes, **caractérisé en ce que** le solvant peut être séparé du milieu réactionnel avant ou après addition de l'acide.

## Patentansprüche

1. Verfahren zur Herstellung von Aminonitril und Diamin durch katalytische Hydrierung von aliphatischem Dinitril mit 3 bis 12 Kohlenstoffatomen, gegebenenfalls in Anwesenheit von Lösungsmittel, **dadurch gekennzeichnet, daß** die am Schluß erhaltene Reaktionsmischung, deren Katalysator zuvor abgetrennt wurde, durch Zugabe einer ausreichenden Menge einer Mineralsäure oder organischen Säure angesäuert wird, bevor man die Reaktionsprodukte und das nicht umgewandelte Dinitril durch Destillation extrahiert.

2. Verfahren nach Anspruch 1, **dadurch gekennzeichnet, daß** es bei Mischungen angewendet wird, die aus der Hydrierung von Nitrilen stammen, katalysiert durch mindestens ein Metall der Gruppe VIII des Periodensystems der Elemente.

3. Verfahren nach einem der Ansprüche 1 oder 2, **dadurch gekennzeichnet, daß** es bei Reaktionsmischungen angewendet wird, die aus der Herstellung von 6-Aminocapronitril und Hexamethylendiamin durch katalytische Hydrierung von Adiponitril stammen.

4. Verfahren nach einem der Ansprüche 1 bis 3, **dadurch gekennzeichnet, daß** die Hydrierung in Anwesenheit von Katalysatoren durchgeführt wird, die mindestens ein Metall umfassen, ausgewählt unter Nickel, Cobalt, Eisen, Rhodium, Ruthenium, gegebenenfalls assoziiert mit einem oder mehreren Promotorelementen.

5. Verfahren nach Anspruch 4, **dadurch gekennzeichnet, daß** die Promotorelemente ausgewählt werden unter Molybdän, Wolfram, Titan, Chrom, Eisen, Nickel, Cobalt, Kupfer, Silber, Gold, Zink, Cadmium, Blei, Zinn, Palladium, Platin, Osmium, Rhenium, Iridium, Antimon, Wismut und den Metallen der Seltenen Erden.

6. Verfahren nach einem der Ansprüche 1 bis 5, **dadurch gekennzeichnet, daß** der Katalysator auf einem Träger angeordnet ist, ausgewählt unter Aluminiumoxid, Siliciumdioxid, Titandioxid, Zirkoniumdioxid, Magnesiumoxid und den Aktivkohlen.

7. Verfahren nach einem der Ansprüche 1 bis 5, **dadurch gekennzeichnet, daß** der Katalysator ausgewählt wird unter Raney-Nickel und Raney-Cobalt, gegebenenfalls umfassend ein oder mehrere Promotorelemente wie Titan, Molybdän, Chrom, Eisen, Wolfram, Zink, Kupfer, Silber und Gold.

8. Verfahren nach einem der Ansprüche 1 bis 5, **dadurch gekennzeichnet, daß** der Katalysator ausgewählt wird unter den Katalysatoren auf der Basis von Nickel oder Cobalt, mindestens teilweise im reduzierten Zustand und texturiert durch eine Phase, die ein oder mehrere Promotormetalle in Form von Oxiden umfaßt.

9. Verfahren nach einem der Ansprüche 1 bis 8, **dadurch gekennzeichnet, daß** die Säure, die der Reaktionsmischung nach der Abtrennung des Katalysators zugesetzt wird, eine Mineralsäure wie Schwefelsäure, Phosphorsäure, phosphorige Säure, Chlorwasserstoffsäure, Salpetersäure, oder eine organische Säure, wie die aliphatischen, cycloaliphatischen, aromatischen, mono- oder polyfunktionellen Carbonsäuren, oder die aliphatischen, cycloaliphatischen oder aromatischen Sulfonsäuren, oder ein saures Harz ist, vorzugsweise mit Sulfongruppen.

10. Verfahren nach einem der Ansprüche 1 bis 9, **dadurch gekennzeichnet, daß** die Menge von eingesetzter Säure derart ist, daß sie mindestens der Stöchiometrie entspricht, bezogen auf die mineralischen basischen Verbindungen, die in der aus der Hydrierung von Dinitril stammenden Reaktionsmischung anwesend sind.

11. Verfahren nach einem der Ansprüche 1 bis 10, **dadurch gekennzeichnet, daß** die Menge von eingesetzter Säure 0,0005 Gew.-% bis 2 Gew.-% Säure, bezogen auf das Gewicht der Reaktionsmischung, und vorzugsweise 0,001 Gew.-% bis 1 Gew.-% pro Gewicht ausmacht.

12. Verfahren nach einem der vorstehenden Ansprüche, **dadurch gekennzeichnet, daß** das Lösungsmittel von dem Reaktionsmedium vor oder nach der Zugabe der Säure abgetrennt wird.

## Claims

1. Process for the preparation of aminonitrile and of diamine by catalytic hydrogenation of aliphatic dinitrile having from 3 to 12 carbon atoms, optionally in the presence of a solvent, **characterized in that** the final reaction mixture, the catalyst of which has been separated beforehand, is acidified by addition of a sufficient amount of an inorganic or organic acid, before extracting, by distillation, the products of the reaction and the unconverted dinitrile.

2. Process according to Claim 1, **characterized in that** it applies to the mixtures originating from the hydrogenation of dinitriles which is catalysed by at least one metal from group VIII of the Periodic Classification of the Elements.

3. Process according to either of Claims 1 and 2, **characterized in that** it is applied to the reaction mixtures originating from the preparation of 6-aminocapronitrile and of hexamethylenediamine by catalytic hydrogenation of adiponitrile.

4. Process according to one of Claims 1 to 3, **characterized in that** the hydrogenation is carried out in the presence of catalysts comprising at least one metal chosen from nickel, cobalt, iron, rhodium or ruthenium, optionally in combination with one or more promoter elements.

5. Process according to Claim 4, **characterized in that** the promoter elements are chosen from molybdenum, tungsten, titanium, chromium, iron, nickel, cobalt, copper, silver, gold, zinc, cadmium, lead, tin, palladium, platinum, osmium, rhenium, iridium, antimony, bismuth or rare earth metals.

6. Process according to one of Claims 1 to 5, **characterized in that** the catalyst is deposited on a support chosen from alumina, silica, titanium dioxide, zirconium dioxide, magnesium oxide or active charcoals.

7. Process according to one of Claims 1 to 5, **characterized in that** the catalyst is chosen from Raney nickel and Raney cobalt, optionally comprising one or more promoter elements, such as titanium, molybdenum, chromium, iron, tungsten, zinc, copper, silver or gold.

8. Process according to one of Claims 1 to 5, **characterized in that** the catalyst is chosen from catalysts based on nickel or on cobalt at least partially in the reduced state, which metal is texturized by a phase comprising one or more promoter metals in the form of oxides.

9. Process according to one of Claims 1 to 8, **characterized in that** the acid added to the reaction mixture after separation of the catalyst is an inorganic acid, such as sulphuric acid, phosphoric acid, phosphorous acid, hydrochloric acid or nitric acid, or an organic acid, such as aliphatic, cycloaliphatic or aromatic carboxylic acids, which can be mono- or polyfunctional, or aliphatic, cycloaliphatic or aromatic sulphonic acids, or an acidic resin, preferably comprising sulpho groups.

10. Process according to one of Claims 1 to 9, **characterized in that** the amount of acid employed is such that it corresponds at least to stoichiometry with respect to the basic inorganic compounds present in the reaction mixture resulting from the hydrogenation of the dinitrile.

11. Process according to one of Claims 1 to 10, **characterized in that** the amount of acid employed represents from 0.0005% to 2% by weight of acid with respect to the weight of the reaction mixture and preferably from 0.001% to 1% as weight by weight.

12. Process according to one of the preceding claims, **characterized in that** the solvent can be separated from the reaction mixture before or after addition of the acid.
